# EUROPEAN PATENT SPECIFICATION

(11) **EP 0 474 650 B1**
(45) Date of publication and mention of the grant of the patent: **05.04.1995**
(21) Application number: 90906825.6
(22) Date of filing: 18.05.1990
(51) Int. Cl.: A61B 5/03, A61B 5/11

(54) **DOLORIMETER APPARATUS**
GERÄT ZUR ANZEIGE DER SCHMERZEMPFINDUNG
APPAREIL DE DOLORIMETRIE

(30) Priority: 19.05.1989 US 354370
(43) Date of publication of application: 18.03.1992
(73) Proprietor: The University of Victoria Innovation and Development Corporation, Victoria, British Columbia V8W 2Y2 (CA)
(72) Inventor: ZIELINSKI, Adam, Victoria, British Columbia V8N 5N3 (CA); ATKINS, Christopher, John, Victoria, British Columbia V8N 1L2 (CA)
(74) Representative: Gordon, Michael Vincent
(86) International application number: PCT/CA90/00163
(87) International publication number: WO 90/14042

(56) References cited:
- AU-A- 516 026
- DE-U- 8 708 251
- US-A- 4 768 521
- IEEE TRANSACTIONS ON BIO-MEDICAL ENGINEERING vol. MBE35, no. 12, December 1988, NEW YORK US pages 1090-1093; J. SORAB et al.: "Tactile Sensory Monitoring of Clinician-Applied Forces During Delivery of Newborns" see pages 1090-1092; figures 2-4 "Abstract" and section II. "System Design and Construction"
- "IEEE 9th Ann. Conf. of the Engineering in Medicine and Biology Society" 16 November 1987, IEEE, Boston, MA (US) see pages 1757 - 1758; figures 1, 3

## Description

This invention relates to an apparatus for measuring pressure applied to a patient, for example the minimum pressure applied to an arthritic joint which elicits discomfort.

Physicians place an important role on patterns of pain in the diagnosis and management of their patients. Manual palpation is the standard method of examination, but it has a certain drawback, namely that the procedure is subjective and lacks the precision necessary to accurately assess, for example, the degree of inflammation of arthritic patients.

The limitations of manual palpation have been addressed by providing mechanical devices known as dolorimeters, algesimeters or algometers (the terms are used synonymously herein). In the simplest form, a mechanical dolorimeter includes a simple spring-loaded probe connected to a gauge. The gauge indicates the deflection of the probe and hence the pressure applied to the probe. In use, the physician presses the probe against the inflamed joint or other portion of the patient's body suffering pain, and applies pressure until the patient feels discomfort. The reading of the gauge is noted, the reading being an objective indication of the degree of inflammation of the joint, for example.

Electronic dolorimeters have been developed, such as disclosed in US-A-4641661. That device includes an electronic circuit housed in a hand-held unit. The dolorimeter has a probe with a resistance which varies according to pressure applied to the probe. The hand-held unit is capable of measuring the resistance of the probe and thereby the pressure applied.

Other devices for determining or recording applied pressure, pain sensitivity or the like are disclosed in US-A-4144877, US-A-4501148, US-A-4503705, US-A-4768521, USSR-A-166999, DE-C-230696 and EP-B-0158336.

It is also known for tactile-sensing systems to include finger-mounted sensors. An arrangement having a piezoresistive sensor encapsulated in a casing of silicone gel is disclosed in the IEEE Transactions on Biomedical Engineering; vol. BME35, no. 12, 1988, pages 1090-1093; J. Sorab et al;: "Tactile sensory monitoring of clinician applied forces during delivery of newborns". That document has been acknowledged by the pre-characterising portion of the present independent claim. A similar arrangement, wherein a deformable electrode is mounted over a rigid ceramic substrate, is disclosed in the IEEE 9th Ann. Conf. of the Engineering in Medicine and Biology Society; 16.11.87; IEEE Boston; pages 1757-1758; K.M. Rutherford et al;: "A finger mounted force sensor for use with an FNS hand grasp system".

The devices above substitute the finger of the physician with an inanimate probe, or provide relatively thick or relatively hard force sensors. For this reason, they have an inherent drawback in that they remove certain advantages to the physician and the patient inherent in the touch of the physician's finger. The physician's finger is capable of determining with accuracy the precise location on body tissue where the pain threshold is to be assessed. It is not always easy for the physician to press the inanimate probe at precisely the right location because he or she receives no direct tactile feedback from the probe. In addition, there is an impersonal aspect objectionable to some patients associated with the act of being pressed with an inanimate object. Many patients would prefer the more personal contact of a physician's finger. Prior art force sensors which are relatively thick or relatively hard detract from the physician's ability to locate precisely areas for palpation.

An object of the present invention is to reduce the difficulties of the prior art by providing a dolorimeter apparatus which can not only output an objective reading of the minimum pressure which causes discomfort but also provide the beneficial aspects of manual palpation by enabling a physician to locate easily and precisely an area for palpation.

According to the present invention, a dolorimeter apparatus is provided comprising:
a pressure responsive member having an electrical property which varies with pressure applied to the member, the member being dimensioned to fit between a finger tip of a user and body tissue to be touched by the user; securing means for securing the member under the finger tip of the user of the apparatus; and detecting means for detecting variance in the electrical property of the member, the detecting means communicating with the pressure responsive member; characterised in that:
(a) the pressure responsive member is flexible and comprises a pressure sensitive film and a flexible substrate, the film being carried on the substrate, the member being sufficiently thin and flexible to permit substantial tactile communication between the finger tip and the body tissue by permitting the finger tip to determine accurately a precise location on the tissue; and
(b) the securing means is a band of a size to pass around the finger for securing the member to the finger.

Preferably, the property which varies is electrical resistance of the film, and the detecting means for detecting variance includes a means for quantifying variance in said property of the member and for displaying a value indicative of said property, and thereby the pressure applied to the member.

The means for quantifying and displaying may include an ohmeter.

A dolorimeter apparatus, in accordance with the present invention, will now be described in greater detail with reference to the accompanying drawings, in which:-
Fig. 1 is a perspective view showing a dolorimeter apparatus including a pressure responsive member secured to a finger of a user, a unit mounted on the wrist of the user for receiving a signal from the pressure responsive member and for displaying indicia representing said pressure, and a finger of a patient;
Fig. 2 is an elevation of the pressure responsive member removed from the finger, showing some detail and one means of fastening the member to the finger; and
Fig. 3 is an electronic block diagram of the dolorimeter apparatus.

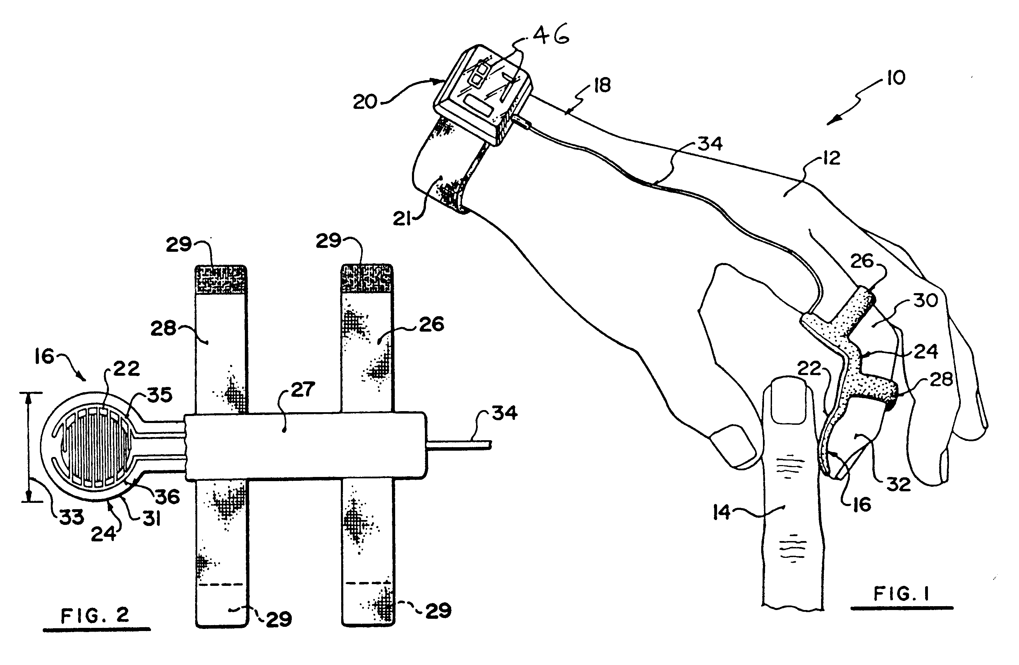

Referring to Figs. 1 and 2, a dolorimeter apparatus is shown generally at 10 in association with a physician's hand 12 and a finger 14 of a patient.

The apparatus has two principal components, namely a flexible pressure responsive member 16 having an electrical property which varies according to pressure applied to the member, and a receiving and display apparatus 20. The member 16 is attached to a physician's index finger 30 and, in this preferred example, the apparatus 20 is mounted on wrist 18 of the physician using a wrist strap 21.

Referring firstly to the pressure responsive member 16, the member includes a pressure sensitive film 22 on a flexible, sheet-like substrate 24. In the preferred embodiment, the film 22 is a shunt-mode force sensing resistor of the type sold by Interlink Electronics of Santa Barbara California, U.S.A. This device has an electrical resistivity which varies according to the pressure applied to the film. The film 22 is mounted on a substrate which may be, for example, a relatively thin elastomeric membrane having contacts printed thereon for contacting the film 22. The member further includes securing means for securing the member to the physician's hand which comprises a pair of straps or bands 26 and 28 in this embodiment, the bands being shaped to fit about the index finger 30 of the physician. The straps are connected to a proximal portion 27 of the member 16. Ends of the straps can be releasably connected together to form the bands using releasable connecting means such as "Velcro" fasteners 29, Velcro being a trade mark of the Velcro Corporation. When fitted in place, the film 22 is under tip 32 of the index finger. Also the film has an overall size that approximates to a sensitive portion of the finger tip that is used by the physician for manual palpation. It is important that the pressure responsive member interferes negligibly with normal palpation, and thus it must have a thickness and flexibility that will permit the finger tip of the examining physician to navigate the intricacies of tissues being palpated, for example arthritic finger joints, etc. During normal use, the member 16 will bend when pressed by the finger tip against hard tissue which is surrounded by soft tissue, and the bending of the member 16 should have a negligible effect on the output therefrom. The output characteristics of the member 16 should be such that only actually applied pressure, causing compressive deformation of the member 16, should effect the output of the member and merely bending of the member should have a negligible effect on its output.

Referring to Figure 2, the member 16 has a pressure responsive distal portion 31 which is disposed in a generally circular plan form having a diameter 33 of approximately 0.5 inches (1.0 cms) to approximate to size of the finger tip. The member 16 has a plurality of chordally disposed interdigitated conductors extending alternately from arcuate peripheral conductors 35 and 36 disposed on opposite sides thereof. The member 16 has an overall thickness preferably of between 10 - 20 mil (0.254 - 0.508 mms), and a flexibility that permits bending under normal forces generated during manual palpation. Typically, the film 22 of the shunt mode device is printed on a substrate 24 of Mylar (trade mark) having a thickness of between 3 and 7 mil (0.0762 - 0.1778 mms). Force sensing resistors as manufactured by Interlink Electronics are manufactured in accordance with U.S. Patent 4,314,228. It can be seen that the sensor is very thin and essentially uniformly flexible to interfere minimally with normal palpation.

The apparatus includes means for outputting a signal representing the property of the member which varies with pressure applied to the member, in this case the resistivity of the film. In this embodiment the means for outputting includes a 2-wire electrical conductor 34 which is connected to the contacts contacting the film 22 at one end and to receiving and display apparatus 20 at the other end.

The receiving and display apparatus in its simplest form can be an ohmmeter with digital display 46 on the face thereof for indicating the instantaneous resistance of the film. Such ohmmeters are well known and thus an additional description is not provided. The indicia can simply be a numerical representation, for example on a liquid crystal display, of the resistance of the film. Preferably, however, the apparatus 20 includes a circuit for converting the resistance value to a number in units meaningful to the physician indicative of the pressure applied to finger 14, such as psi or new units unique to this instrument.

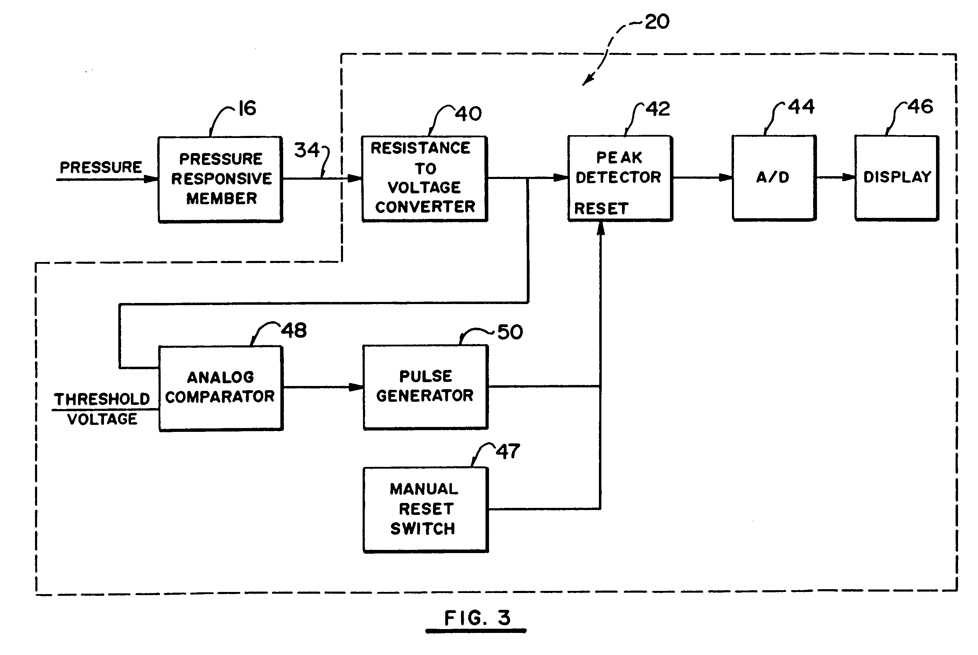

Referring to Figure 3, in the preferred embodiment the pressure responsive member 16 is connected by the electrical conductor 34 to a resistance-to-voltage converter 40 of the display apparatus shown in broken outline generally at 20. The resistance-to-voltage converter provides an output voltage signal representing resistance measured at the pressure responsive member and therefore ultimately represents the pressure applied to the member.

The output of the resistance-to-voltage converter is supplied to a peak detector 42. The peak detector monitors the output voltage from the resistance-to-voltage converter. The peak detector provides its own output voltage which tracks the output voltage of the resistance-to-voltage converter until a highest value is attained, at which time the output voltage of the peak detector is held at this highest value. The output voltage from the peak detector is supplied to an analog-to-digital converter 44 where it is converted into a digital format. The digital format is supplied to an output device such as a digital display 46 which displays data representing pressure applied at the pressure responsive member. Thus, the electronic circuit has means for tracking said signal and for automatically holding said signal in a state representing a highest value of pressure applied to the member.

The display device has two modes of operation, one in which the device is manually reset and a second in which the device is automatically reset. In the manual reset mode, a simple switch 47 is provided to reset the peak detector 42 after a pressure measurement is taken. To use the device in this mode, a physician applies pressure to a patient's finger through the pressure responsive member 16. As the physician gradually increases pressure on the finger, the output voltage of the resistance-to-voltage converter 40 is monitored by the peak detector 42 and pressure values are seen to increase at the digital display 46. At the onset of pain indicated by the patient, the physician may release the pressure from the finger, and thus also from the member 16, at which time the peak detector 42 will retain at its output the voltage representing the highest pressure inflicted or, in other words, the pain threshold of the patient. The pain threshold pressure value will be displayed on the digital display 46. The display 46 will continue to indicate this pressure value until the physician actuates the manual reset switch thereby resetting the peak detector 42. Resetting the peak detector 42 sets the display 46 back to zero. The apparatus is thus rendered ready to take another pressure measurement.

An analog comparator 48 and a pulse generator 50 are incorporated into the circuit for the automatic reset capability. In the automatic reset mode, the output of the resistance-to-voltage converter is supplied to the analog comparator 48. The comparator compares this voltage with a threshold voltage level and causes the output of the comparator to change the state of its output when the threshold level is exceeded. The output of the comparator is connected to the pulse generator 50 which detects the change in state of the comparator and generates a pulse of short duration. The pulse is supplied to the peak detector 42 and serves to reset the peak detector when the voltage from the resistance-to-voltage converter crosses the threshold level at the comparator. Typically, the threshold level at the comparator is set to an amount lower than the minimum voltage output of the resistance-to-voltage converter for a minimum pressure reading taken by the physician.

In operation, in the automatic reset mode, the physician gradually applies pressure to the finger of the patient through the member 16, thereby changing the resistance of the pressure responsive member and causing the output of the resistance-to-voltage converter 40 to increase. Upon application of minimal pressure, the output voltage of the resistance-to-voltage converter 40 exceeds the threshold voltage level at the comparator and causes the comparator output to change state. This change of state is detected by the pulse generator 50 which resets the peak detector 42. The peak detector 42 is thus rendered ready to track and monitor the output of the resistance-to-voltage converter. The digital display 46 continuously displays the changing output voltage of the peak detector as the pressure on the patient's finger is increased.

At the onset of pain indicated by the patient, the physician releases the pressure on the patient's finger and the peak detector maintains its output voltage at a value representing the greatest pressure applied to the patient. A numerical value representing this pressure is indicated in appropriate units and maintained on the display even after the physician has released the pressure on the patient's finger. The numerical value will continue to be displayed until pressure is again applied to the pressure responsive member which generates a voltage above the threshold level to reset the peak detector. It may readily be seen that when using the device in the automatic mode, the physician need not press the manual reset button and therefore the device can be easily operated with one hand only. Use of the device in the automatic reset mode also appreciably reduces examination time.

Other pressure responsive means could be used for receiving and displaying the value indicative of the pressure applied to the body tissue provided the means has a known output characteristic which is constant in response to a constant or statically applied compressive force. Other means can be used to detect the variance in the electrical property, for example, a Wheatstone bridge can be used to determine the resistance of the pressure responsive film. In addition, the member 16 can have other variable electrical properties dependent upon applied pressure. For example a member with a variable capacitance property is another possibility. Such a sensor can be formed by depositing thin conducting layers on both sides of a thin elastomeric substrate. A pressure applied to such material will change its thickness and therefore capacitance of the sensor. Changes of capacitance can be sensed by a variety of well known methods such as an AC bridge, or a variable frequency oscillator.

The member 16, and in particular the portion under the finger tip 32 is sized so as to permit substantial tactile communication to the physician's finger when touching an object, such as the joint of the patient's finger 14. In this preferred embodiment the tactile communication is achieved by making the film and the substrate relatively thin and flexible to accommodate the sense of the touch of the physician. It can be appreciated that the invention is not a substitute for the human finger tip, which is, by itself, a very sensitive diagnostic device. The invention fits adjacent the finger tip and does not interfere appreciably with the physician's ability to use the finger tip to determine with accuracy a precise location on body tissue where pressure is to be applied. The invention also permits the finger to apply pressure in a normal manner. In addition, the invention provides an accurate output of the actual applied pressure, and thus works in concert with the finger tip. The invention reduces or eliminates the need for the physician to try to ascertain, subjectively, the force applied to the joint by the finger tip. It is added that the invention permits the physician to detect many other tissue characteristics of the arthritic joint, such as tissue tension, tissue heat and consistency of the tissues of the joint being palpated, which are normally detectable. The invention exhibits relatively high acuity and permits accurate reproduction of the applied force.

In another version of the invention, there is a communications ability between receiving and display apparatus 20 and a computer to record measured data. A short range telemetry link, such as an infrared beam similar to that used for T.V. controls, can be employed.

While specific embodiments of the invention have been described, such embodiments should not be considered as limiting the scope of the invention as construed in accordance with the accompanying claims.

## Claims

1. A dolorimeter apparatus (10) comprising:
a pressure responsive member (16) having an electrical property which varies with pressure applied to the member, the member being dimensioned to fit between a finger tip (32) of a user and body tissue (14) to be touched by the user; securing means (26, 28) for securing the member under the finger tip (32) of the user of the apparatus; and detecting means (20) for detecting variance in the electrical property of the member, the detecting means communicating with the pressure responsive member (16); characterised in that:
(a) the pressure responsive member (16) is flexible and comprises a pressure sensitive film (22) and a flexible substrate (24), the film being carried on the substrate, the member being sufficiently thin and flexible to permit substantial tactile communication between the finger tip and the body tissue by permitting the finger tip to determine accurately a precise location on the tissue; and
(b) the securing means is a band (26, 28) of a size to pass around the finger for securing the member (16) to the finger.

2. An apparatus as claimed in claim 1, characterised in that the flexible substrate (24) has a thickness of between approximately 0.076 millimetres and 0.178 millimetres.

3. An apparatus as claimed in claim 1 or claim 2, characterised in that the pressure responsive member (16) has an overall thickness of between approximately 0.25 millimetres and 0.51 millimetres.

4. An apparatus as claimed in any one of claims 1 to 3, characterised in that said electrical property is electrical resistance of the film.

5. An apparatus as claimed in any one of claims 1 to 3, characterised in that said electrical property is electrical capacitance.

6. An apparatus as claimed in any preceding claim, characterised in that the detecting means (20) for detecting variance in the electrical property is connected to the member (16) by an electrical conductor (34).

7. An apparatus as claimed in any preceding claim, characterised in that the detecting means (20) for detecting variance includes a means for quantifying variance (40, 42) in said electrical property.

8. An apparatus as claimed in claim 7, characterised in that the means for quantifying the variance in said electrical property of said member includes electronic circuit means (20) for providing a signal representing the pressure applied to the member (16).

9. An apparatus as claimed in claim 8, characterised in that said electronic circuit means includes means (20) for tracking said signal and for automatically holding said signal in a state representing a highest value pressure applied to the member (16).

10. An apparatus as claimed in claim 9, characterised in that the means for tracking and holding is automatically reset when said signal crosses the threshold value.

11. An apparatus as claimed in claim 8, characterised in that said electronic circuit means (20) includes an output device (46) for indicating pressure applied to the member.

12. An apparatus as claimed in claim 11, characterised in that said output device includes a digital display (46).

13. An apparatus as claimed in any preceding claims, characterised in that the pressure responsive member (16) has a characteristic such that bending of the member as encountered during normal palpation has a negligible effect on output from the member.

## Patentansprüche

1. Gerät (10) zur Schmerzempfindungsanzeige, mit: einem druckempfindlichen Teil (16) mit einer elektrischen Eigenschaft, die sich mit dem auf das Teil ausgeübten Drucken verändert, wobei das Teil so bemessen ist, daß es zwischen eine Fingerspitze (32) eines Benutzers und ein Körpergewebe (14) paßt, das von dem Benutzer zu berühren ist; Befestigungsmittel (26, 28) zum Befestigen des Teiles unter der Fingerspitze (32) des Benutzers des Gerätes; und Erfassungsmittel (20) zum Erfassen einer Veränderung der elektrischen Eigenschaft des Teiles, wobei das Erfassungsmittel mit dem druckempfindlichen Tei in Verbindung steht; dadurch gekennzeichnet, daß
(a) das druckempfindliche Teil (16) flexibel ist und einen druckempfindlichen Film (22) und ein flexibles Substrat (24) aufweist, wobei der Film auf dem Substrat getragen ist, das Teil ausreichend dünn und flexibel ist, so daß eine im wesentlichen taktile Verbindung zwischen der Fingerspitze und dem Körpergewebe möglich ist, indem der Fingerspitze ermöglicht wird, genau eine präzise Stelle auf dem Gewebe zu bestimmen; und
(b) das Befestigungsmittel ein Band (26, 28) einer derartigen Größe ist, daß es um den Finger geht zum Befestigen des Teiles (16) an dem Finger.

2. Gerät nach Anspruch 1, dadurch gekennzeichnet, daß das flexible Substrat (24) eine Dicke von zwischen ungefähr 0,076 mm und 0,178 mm aufweist.

3. Gerät nach Anspruch 1 oder Anspruch 2, dadurch gekennzeichnet, daß das druckempfindliche Teil (16) eine Gesamtdicke von zwischen ungefähr 0,25 mm und 0,51 mm aufweist.

4. Gerät nach einem der Ansprüche 1 bis 3, dadurch gekennzeichnet, daß die elektrische Eigenschaft der elektrische Widerstand des Filmes ist.

5. Gerät nach einem der Ansprüche 1 bis 3, dadurch gekennzeichnet, daß die elektrische Eigenschaft die elektrische Kapazität ist.

6. Gerät nach einem der vorhergehenden Ansprüche, dadurch gekennzeichnet, daß das Erfassungsmittel (20) zum Erfassen der Veränderung in der elektrischen Eigenschaft mit dem Teil (16) durch einen elektrischen Leiter (34) verbunden ist.

7. Gerät nach einem der vorhergehenden Ansprüche, dadurch gekennzeichnet, daß das Erfassungsmittel (20) zum Erfassen der Veränderung ein Mittel zum Quantifizieren der Veränderung (40, 42) der elektrischen Eigenschaft enthält.

8. Gerät nach Anspruch 7, dadurch gekennzeichnet, daß das Mittel zum Quantifizieren der Veränderung der elektrischen Eigenschaft des Teiles ein elektronisches Schaltungsmittel (20) zum Vorsehen eines Signales ist, das den auf das Teil (16) ausgeübten Druck darstellt.

9. Gerät nach Anspruch 8, dadurch gekennzeichnet, daß das elektronische Schaltungsmittel ein Mittel (20) zum Verfolgen des Signales und zum automatischen Halten des Signales in einem Zustand enthält, das einen höchsten Druckwert darstellt, der auf das Teil (16) ausgeübt ist.

10. Gerät nach Anspruch 9, dadurch gekennzeichnet, daß das Mittel zum Verfolgen und Halten automatisch zurückgesetzt wird, wenn das Signal den Schwellenwert überschreitet.

11. Gerät nach Anspruch 8, dadurch gekennzeichnet, daß das elektronische Schaltungsmittel (20) eine Ausgabeeinrichtung (46) zum Anzeigen des auf das Teil ausgeübten Druckes enthält.

12. Gerät nach Anspruch 11, dadurch gekennzeichnet, daß die Ausgabeeinrichtung eine digitale Anzeige (46) enthält.

13. Gerät nach einem der vorhergehenden Ansprüche, dadurch gekennzeichnet, daß das druckempfindliche Teil (16) eine Eigenschaft derart aufweist, das Biegen des Teiles, wie es während normalem Antasten begegnet wird, eine vernachläßigbaren Effekt auf die Ausgabe des Teiles aufweist.

## Revendications

1. Appareil dolorimètre (10), comprenant :
un organe manosensible (16) ayant une propriété électrique qui varie avec la pression appliquée à l'organe, celui-ci ayant des dimensions telles qu'il peut s'ajuster entre le bout (32) d'un doigt d'un utilisateur et un tissu (14) du corps destiné à être touché par l'utilisateur, un dispositif (26, 28) de fixation de l'organe sous le bout (32) du doigt de l'utilisateur de l'appareil, et un dispositif (20) de détection de la variation de la propriété électrique de l'organe, le dispositif de détection communiquant avec l'organe manosensible (16), caractérisé en ce que :
a) l'organe manosensible (16) est souple et comporte un film manosensible (22) et un substrat souple (24), le film étant porté par le substrat, l'organe étant suffisamment mince et souple pour permettre une communication tactile importante entre le bout du doigt et le tissu du corps en permettant au bout du doigt de déterminer avec précision un emplacement précis sur le tissu, et
b) le dispositif de fixation est une bande (26, 28) dont la dimension lui permet de passer autour du doigt pour la fixation de l'organe (16) au doigt.

2. Appareil selon la revendication 1, caractérisé en ce que le substrat souple (24) a une épaisseur comprise entre environ 0,076 et 0,178 mm.

3. Appareil selon la revendication 1 ou 2, caractérisé en ce que l'organe manosensible 16 a une épaisseur totale comprise entre environ 0,25 et 0,51 mm.

4. Appareil selon l'une quelconque des revendications 1 à 3, caractérisé en ce que la propriété électrique est la résistance électrique du film.

5. Appareil selon l'une quelconque des revendications 1 à 3, caractérisé en ce que la propriété électrique est la capacité électrique.

6. Appareil selon l'une quelconque des revendications précédentes, caractérisé en ce que le dispositif (20) de détection de la variation de la propriété électrique est connecté à l'organe (16) par un conducteur électrique (34).

7. Appareil selon l'une quelconque des revendications précédentes, caractérisé en ce que le dispositif (20) de détection de la variation comporte un dispositif de quantification de la variation (40, 42) de la propriété électrique.

8. Appareil selon la revendication 7, caractérisé en ce que le dispositif de quantification de la variation de la propriété électrique de l'organe comprend un dispositif à circuit électronique (20) destiné à donner un signal représentant la pression appliquée à l'organe (16).

9. Appareil selon la revendication 8, caractérisé en ce que le dispositif à circuit électronique comprend un dispositif (20) destiné à suivre le signal et à maintenir automatiquement le signal à un état représentant la pression ayant la plus grande valeur appliquée à l'organe (16).

10. Appareil selon la revendication 9, caractérisé en ce que le dispositif destiné à suivre et maintenir est réarmé automatiquement lorsque le signal recoupe la valeur de seuil.

11. Appareil selon la revendication 8, caractérisé en ce que le dispositif (20) à circuit électronique comprend un dispositif de sortie (46) destiné à indiquer la pression appliquée à l'organe.

12. Appareil selon la revendication 11, caractérisé en ce que le dispositif de sortie comporte un afficheur numérique (46).

13. Appareil selon l'une quelconque des revendications précédentes caractérisé en ce que l'organe manosensible (16) a une caractéristique telle que la flexion de l'organe qui se produit lors d'une palpation normale a un effet négligeable sur le signal de sortie de l'organe.
